# EUROPÄISCHE PATENTSCHRIFT

(11) **EP 2 114 938 B1**
(45) Veröffentlichungstag und Bekanntmachung des Hinweises auf die Patenterteilung: **06.08.2014**
(21) Anmeldenummer: 08708306.9
(22) Anmeldetag: 28.01.2008
(51) Int. Cl.: C07D 451/06

(54) **VERFAHREN ZUR HERSTELLUNG VON SCOPINIUMSALZEN**
METHOD FOR PRODUCING SCOPINIUM SALTS
PROCÉDÉ DE PRODUCTION DE SELS DE SCOPINIUM

(30) Priorität: 29.01.2007 EP 07101363
(43) Veröffentlichungstag der Anmeldung: 11.11.2009
(73) Patentinhaber: Boehringer Ingelheim Pharma GmbH & Co. KG, 55216 Ingelheim am Rhein (DE)
(72) Erfinder: BRANDENBURG, Jörg, 55216 Ingelheim am Rhein (DE); BELZER, Werner, 55216 Ingelheim am Rhein (DE)
(74) Vertreter: Simon, Elke Anna Maria
(86) Internationale Anmeldenummer: PCT/EP2008/050988
(87) Internationale Veröffentlichungsnummer: WO 2008/092833

(56) Entgegenhaltungen:
- WO-A-2007/012626
- J. MEINWALD, E. L. CHAPMAN: "The Alkaline Hydrolysis of Scopolamine Methoxymethochloride: A New Route to Scopine" JOURNAL OF THE AMERICAN CHEMICAL SOCIETY, Bd. 79, 1957, Seiten 665-666, XP002450914
- R. B. MOFFET, E. R. GARRETT: "Alkaline Hydrolysis of Scopolamine Methyl Bromide and Other Ester of Quaternary Amino Alcohols" JOURNAL OF THE AMERICAN CHEMICAL SOCIETEY, Bd. 77, 1955, Seiten 1245-1248, XP002450915

## Beschreibung

Die Erfindung betrifft ein neues Verfahren zur Herstellung von Scopiniumsalzen der allgemeinen Formel **1** 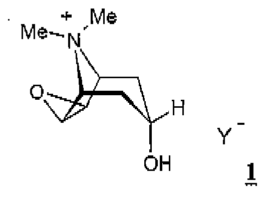 worin Y⁻ die in den Ansprüchen und in der Beschreibung genannten Bedeutungen haben kann.

J. Meinwald, E. L. Chapman, J. Am. Chem. Soc. 79, 1957, pp 665-666 and R.B. Moffet, E.R. Garret, J. Am. Chem. Soc. 77, 1955, pp 1245-1248 offenbaren Verfahren zur Herstellung von Scopinen durch Scopolaminen.

### Beschreibung der Erfindung

Die vorliegende Erfindung betrifft ein Verfahren zur Herstellung von Scopiniumsalzen der Formel **1** 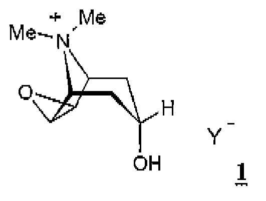 worin
- Y⁻: ein einfach negativ geladenes lipophiles Anion, ausgewählt aus der Gruppe bestehend aus Hexafluorophosphat, Tetrafluoroboranat, Tetraphenylboranat und Saccharinat, bevorzugt Hexafluorophosphat oder Tetraphenylboranat
dadurch gekennzeichnet, daß eine Verbindung der Formel **2** 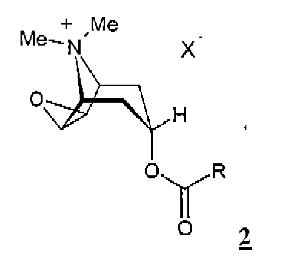 worin
- X⁻: ein einfach negativ geladenes Anion ausgewählt aus der Gruppe bestehend aus Chlorid, Bromid, Iodid, Methansulfonat, p-Toluolsulfonat, Nitrat und Trifluormethansulfonat, bevorzugt Chlorid, Bromid, Iodid, Methansulfonat, Nitrat oder Trifluormethansulfonat, besonders bevorzugt Chlorid, Bromid oder Methansulfonat, besonders bevorzugt Bromid; und
- R: ein Rest ausgewählt aus C₁-C₄-Alkyl, C₂-C₆-Alkenyl und C₁-C₄-alkylen-Phenyl, die jeweils substituiert sein können durch Hydroxy, Hydroxymethyl oder C₁-C₄-Alkoxy, bedeuten,
gegebenenfalls in Form ihrer Solvate oder Hydrate in einem geeigneten Lösemittel unter Zusatz einer geeigneten Base zunächst zu einer Verbindung der Formel **3** 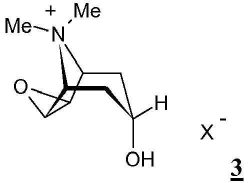 worin X⁻ die vorstehend genannten Bedeutungen haben kann, verseift wird und die Verbindung der Formel **3** ohne Isolierung durch Umsetzung mit einem Salz Kat⁺Y⁻, wobei Kat⁺ für ein Kation ausgewählt aus der Gruppe bestehend aus Li⁺, Na⁺, K⁺, Mg2+, Ca2+, steht und Y⁻ die vorstehend genannten Bedeutungen haben kann, in die Verbindung der Formel **1** überführt wird.

Ein erfindungsgemäß besonders bevorzugtes Verfahren ist dadurch gekennzeichnet, dass die Umsetzung mit einer Verbindung der Formel **2** erfolgt, in der
- R: ein Rest ausgewählt aus -CH₃, -CH₂-CH₃, -CH₂-CH₂-OH, -CH(OH)-CH₃, -CH₂-Phenyl, -CH(OH)-Phenyl und -CH(CH₂OH)-Phenyl, bevorzugt -CH₃, -CH₂-CH₃, -CH₂-Phenyl, und -CH(CH₂OH)-Phenyl, besonders bevorzugt -CH(CH₂OH)-Phenyl, bedeutet.

Besonders bevorzugt ist ein Verfahren mit einer Verbindung der Formel **2**, in der X-Bromid und R -CH(CH₂OH)-Phenyl bedeuten.

Ein erfindungsgemäß besonders bevorzugtes Verfahren betrifft die Herstellung einer Verbindung der Formel **1** in der
- Y⁻: ein einfach negativ geladenes Anion ausgewählt aus der Gruppe bestehend aus Hexafluorophosphat, Tetrafluoroboranat und Tetraphenylboranat, bevorzugt Hexafluorophosphat bedeutet.

Ein erfindungsgemäß besonders bevorzugtes Verfahren ist dadurch gekennzeichnet, dass die Umsetzung der Verbindung der Formel **2** zur Verbindung der Formel **1** mit Hilfe eines Salzes KatY erfolgt, in dem Kat⁺ ausgewählt ist aus der Gruppe bestehend aus Li⁺, Na⁺ und K⁺, besonders bevorzugt Na⁺ und K⁺, und in der Y⁻ die vorstehend genannten Bedeutungen haben kann.

Das erfindungsgemäße Verfahren ist unter anderem dadurch gekennzeichnet, dass es in einem einzigen Schritt ohne notwendige Isolierung der Zwischenverbindung der Formel **3** den direkten Zugang zu Salzen der Formel **1** aus Verbindungen der Formel **2** erlaubt.

Als Alkylgruppen sowie Alkylgruppen, welche Bestandteil anderer Reste sind, werden verzweigte und unverzweigte Alkylgruppen mit 1 bis 4 Kohlenstoffatomen bezeichnet. Beispielsweise werden genannt: Methyl, Ethyl, Propyl, Butyl. Sofern nicht anders genannt, sind von den vorstehend genannten Bezeichnungen Propyl, Butyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl.

Als Alkoxy- oder Alkyloxygruppen, werden verzweigte und unverzweigte Alkylgruppen mit 1 bis 4 Kohlenstoffatomen bezeichnet, die über ein Sauerstoffatom verknüpft sind. Beispielsweise werden genannt: Methoxy, Ethoxy, Propoxy, Butoxy. Sofern nicht anders genannt, sind von den vorstehend genannten Bezeichnungen sämtliche der möglichen isomeren Formen umfaßt.

Unter lipophilen Anionen werden erfindungsgemäß solche Anionen verstanden, deren Natrium- oder Kaliumsalze eine Löslichkeit in polaren organischen Lösungsmitteln wie Methanol oder Aceton von > 1 Gew.-% aufweisen.

Als Lösungsmittel zur Durchführung des erfindungsgemäßen Verfahrens kommen bevorzugt polare Lösemittel in Betracht. Bevorzugte Lösemittel sind erfindungsgemäß ausgewählt aus der Gruppe bestehend aus Wasser, Methanol, Ethanol, Propanol und Isopropanol, wobei Wasser und Methanol erfindungsgemäß herausragende Bedeutung zukommt.

Als Base kommen zur Verseifung der Verbindungen der Formel **2** zu den Verbindungen der Formel **3** vorzugsweise anorganische Basen in Betracht. Beispielsweise seien genannt, die Alkali- oder die Erdalkalicarbonate, -hydroxide und -alkoholate. Bevorzugt werden die Carbonate, Hydroxide und Alkoholate in Form Ihrer Lithium-, Natrium- oder Kaliumssalze eingesetzt. Bevorzugte Basen sind ausgewählt aus der Gruppe bestehend aus Natriumcarbonat, Lithiumcarbonat, Kaliumcarbonat, Calciumcarbonat, Natriumhydroxid, Kaliumhydroxid, Natriummethylat, Natriumethylat, Kaliummethylat oder Kaliumethylat. Besonders bevorzugt wird als anorganische Base eines der vorstehend genannten Kalium- oder Natriumsalze eingesetzt, wobei die Verwendung von Kaliumhydroxid oder Natriummethylat erfindungsgemäß besonders bevorzugt ist.

Grundsätzlich können pro Mol eingesetzte Verbindung der Formel **2** bevorzugt equimolare Mengen an Base eingesetzt, wobei die Base gegebenenfalls auch in leichtem Überschuß eingesetzt werden kann. Wird als Lösungsmittel Methanol verwendet kann pro Mol eingesetzte Verbindung der Formel **2** auch weniger Base Verwendung finden. In einem solchen Fall gelingt die Umsetzung beispielsweise auch unter Verwendung von 0.01 bis 0,5 Mol, bevorzugt 0,02 bis 0,3 Mol, besonders bevorzugt 0,04 bis 0,15 Mol Base pro Mol eingesetzte Verbindung der Formel **2**.

Pro Mol eingesetzte Verbindung der Formel **2** werden erfindungsgemäß bevorzugt 1 Mol , bevorzugt 1- 1.5 Mol, gegebenenfalls auch 2-5 Mol des Salzes Kat⁺Y⁻ eingesetzt. Für den Fachmann ist ersichtlich, dass der Einsatz geringerer Mengen an Salz Kat⁺Y⁻ möglich ist, dass dies aber dann nur zu einer partiellen Umsetzung der Verbindung der Formel **2** führen kann. gegebenenfalls werden die Salze Kat⁺Y⁻ im Rahmen der vorliegenden Erfindung auch nur als Salze KatY bezeichnet.

Das erfindungsgemäße Verfahren wird bevorzugt unter milden Reaktionsbedingungen, das heißt bei Temperaturen im Bereich von 10-55°C, besonders bevorzugt 15-50°C, besonders bevorzugt 20-45°C, durchgeführt. Nach vollständiger Zugabe der Salze KatY, teilweise auch schon während der Zugabe, kristallisieren die Verbindungen der Formel **1** aus der Lösung aus. Die erhaltenen Produkte können, falls erforderlich, durch Umkristallisation aus einem der vorstehend genannten Lösemittel gereinigt werden. Die erhaltenen Kristalle werden isoliert und im Vakuum getrocknet.

Salze quaternärer Ammoniumverbindungen , wie beispielsweise diejenigen der Formel **2** oder **3** sind im allgemeinen sehr gut wasser- und alkohollöslich. Sie sind jedoch ausgesprochen schwer löslich in weniger polaren organischen Solventien wie beispielsweise Aceton, Acetonitril, Kohlenwasserstoffen, Halogenkohlenwasserstoffen oder Ethern. Chemische Umsetzungen mit quaternären Ammoniumverbindungen sind deshalb im Grundsatz auf Umsetzungen in Wasser, Alkohol oder stark polaren aprotischen Lösemitteln wie DMF oder NMP beschränkt. Daraus ergeben sich starke Einschränkungen bezüglich der Auswahl von Reaktionspartnern oder deren Abtrennung vom Zielprodukt.

Viele Synthesestrategien scheitern an der Unmöglichkeit oder Schwierigkeit, quaternäre Ammoniumverbindungen aus wässriger oder alkoholischer Lösung von anderen Reaktionskomponenten zu trennen. Mit Hilfe der Anionen der Formel **1** kann dieses Problem gelöst werden. Es gelingt die selektive Fällung oder Kristallisation der quaternären Ammoniumverbindungen der Formel **1** aus Alkoholen oder Wasser durch Umsetzung der Verbindungen **2** mit den entsprechenden Salzen KatY und damit die Isolierung und Reinigung mit regelmäßig guter Ausbeute.

Die Verbindungen **1** ermöglichen aufgrund ihrer sehr guten Löslichkeit und der ausgesprochen hohen Stabilität des Anions eine Vielzahl von Reaktionen in weniger polaren aprotischen Lösungsmitteln und können dort angewendet werden, wo Wasser oder Alkohol stört. Als Beispiel dazu kann die in nachstehendem Schema 1 und auch im experimentellen Teil der vorliegenden Erfindung ausführlich beschriebene Synthese von Tiotropiumsalzen der Formel **4** dienen.

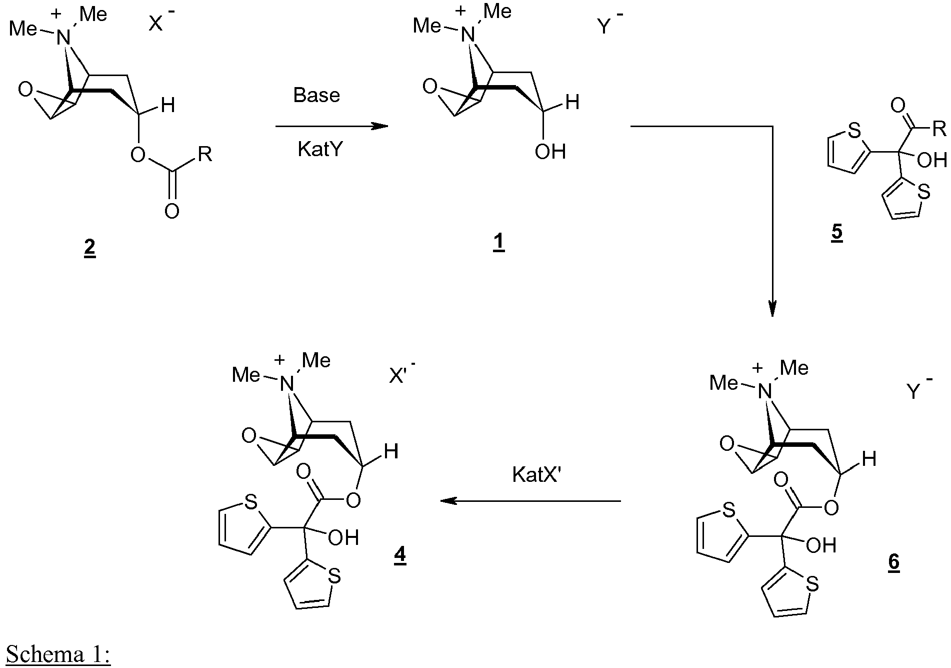

Die vorliegende Erfindung betrifft ferner ein Verfahren zur Herstellung von Tiotropiumsalzen der Formel **4** 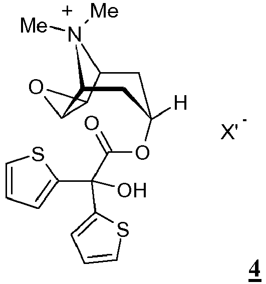 worin
- X'⁻: ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat, p-Toluolsulfonat und Trifluormethansulfonat, bedeuten kann,
dadurch gekennzeichnet, daß eine Verbindung der Formel **2** 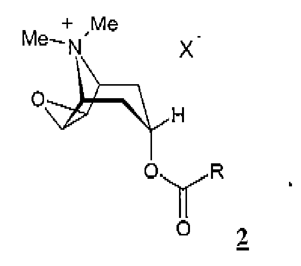 worin
- X⁻: ein einfach negativ geladenes Anion ausgewählt aus der Gruppe bestehend aus Chlorid, Bromid, Iodid, Methansulfonat, p-Toluolsulfonat und Trifluormethansulfonat, bevorzugt Chlorid, Bromid, Iodid, Methansulfonat oder Trifluormethansulfonat, besonders bevorzugt Chlorid, Bromid oder Methansulfonat, besonders bevorzugt Bromid; und
- R: ein Rest ausgewählt aus C₁-C₄-ALkyl, C₂-C₆-Alkenyl und C₁-C₄-alkylen-Phenyl, die jeweils substituiert sein können durch Hydroxy, Hydroxymethyl oder C₁-C₄-Alkoxy, bedeuten,
gegebenenfalls in Form ihrer Säureadditionssalze sowie gegebenenfalls in Form ihrer Hydrate in einem geeigneten Lösemittel unter Zusatz einer geeigneten Base zunächst zu einer Verbindung der Formel **3** 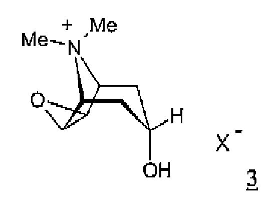 worin X⁻ die vorstehend genannten Bedeutungen haben kann, verseift wird und die Verbindung der Formel **3** ohne Isolierung durch Umsetzung mit einem Salz Kat⁺Y⁻, wobei Kat⁺ für ein Kation ausgewählt aus der Gruppe bestehend aus Li⁺, Na⁺, K⁺, Mg2+, Ca2+, steht und
- Y⁻: ein einfach negativ geladenes lipophiles Anion, ausgewählt ausgewählt aus der Gruppe bestehend aus Hexafluorophosphat, Tetrafluoroboranat, Tetraphenylboranat und Saccharinat, bevorzugt Hexafluorophosphat oder Tetraphenylboranat
bedeuten kann, in die Verbindung der Formel **1** 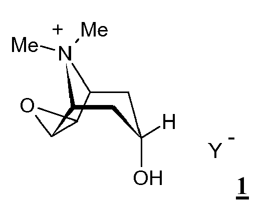 worin Y⁻ die vorstehend genannten Bedeutungen haben kann, überführt wird,
und anschließend die Verbindung der Formel **1** in einem Schritt mit einer Verbindung der Formel **5** 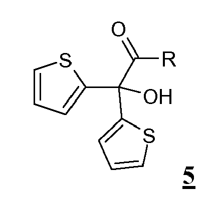 worin
- R: ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet,
in einem geeigneten Lösemittel zu einer Verbindung der Formel **6** 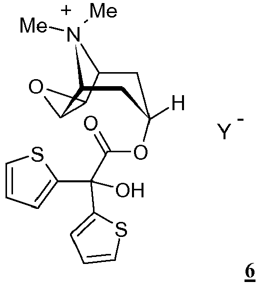 umgesetzt werden, wobei die Gruppe Y⁻ die vorstehend genannten Bedeutungen haben kann, und die Verbindung der Formel **6** ohne Isolierung durch Umsetzung mit einem Salz Kat⁺X'⁻, wobei Kat⁺ für ein Kation ausgewählt aus der Gruppe bestehend aus Li⁺, Na⁺, K⁺, Mg2+, Ca2+, organische Kationen mit quartärem N (z.B. N,N-Dialkylimidazolium, Tetraalkylammonium) steht und X'- die vorstehend genannten Bedeutungen haben kann, in die Verbindung der Formel **4** überführt wird.

Bevorzugt betrifft die vorliegende Erfindung ein Verfahren zur Herstellung von Tiotropiumsalzen der Formel **4** , worin
- X'⁻: ein einfach negativ geladenes Anion ausgewählt aus der Gruppe bestehend aus Chlorid, Bromid, Iodid, Methansulfonat, p-Toluolsulfonat und Trifluormethansulfonat, bevorzugt Chlorid, Bromid, Iodid, Methansulfonat oder Trifluormethansulfonat, besonders bevorzugt Chlorid, Bromid oder Methansulfonat, besonders bevorzugt Bromid, bedeuten kann.

Ein erfindungsgemäß besonders bevorzugtes Verfahren ist dadurch gekennzeichnet, dass die Umsetzung mit einer Verbindung der Formel **5** erfolgt, in der
- R: ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy und 2-Allyloxy bedeutet.

Ein erfindungsgemäß besonders bevorzugtes Verfahren ist dadurch gekennzeichnet, dass die Umsetzung mit einer Verbindung der Formel **5** erfolgt, in der R ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Vinyloxy und 2-Allyloxy, bevorzugt ausgewählt aus Methoxy, Ethoxy, Propoxy, und Butoxy, besonders bevorzugt Methoxy oder Ethoxy bedeutet.

Ein erfindungsgemäß besonders bevorzugtes Verfahren ist dadurch gekennzeichnet, dass die Umsetzung mit einer Verbindung der Formel **1** erfolgt, in der
- Y⁻: ein einfach negativ geladenes Anion ausgewählt aus der Gruppe bestehend aus Hexafluorophosphat, Tetrafluoroboranat und Tetraphenylboranat, bevorzugt Hexafluorophosphat bedeutet.

Das erfindungsgemäße Verfahren ist dadurch gekennzeichnet, dass die abschließende Umsetzung der Verbindung der Formel **6** zur Verbindung der Formel **4** mit Hilfe eines Salzes KatX' erfolgt, in dem Kat⁺ ausgewählt ist aus der Gruppe bestehend aus Li⁺, Na⁺, K⁺, Mg2+, Ca2+, organische Kationen mit quartärem N (z.B. N,N-Dialkylimidazolium, Tetraalkylammonium ) und in der X'- die vorstehend genannten Bedeutungen haben kann.

Das erfindungsgemäße Verfahren zeichnet sich insbesondere dadurch aus, dass es aufgrund der Löslichkeit der Intermediate der Formel **1** und **6** in relativ unpolaren Lösemitteln geführt werden kann. Dies erlaubt eine Umsetzung unter recht schonenden Bedingungen, die im Vergleich zu Reaktionen in hochpolaren aprotischen Lösungsmitteln bei den empfindlichen Tiotropiumsalzen weniger Nebenreaktionen und damit zusammenhängend eine höhere Ausbeute zur Folge haben.

Die Umsetzung der Verbindungen der Formel **1** mit den Verbindungen der Formel **5** wird vorzugsweise in einem aprotischen organischen Lösemittel, bevorzugt in einem schwach polaren organischen Lösemittel durchgeführt. Als Lösemittel kommen erfindungsgemäß besonderes bevorzugt in Betracht Aceton, Pyridin, Acetonitril und Methylethylketon, wobei Aceton, Acetonitril und Pyridin bevorzugt zum Einsatz gelangen. Besonders bevorzugt erfolgt die Umsetzung in einem Lösemittel ausgewählt aus der Gruppe bestehend aus Aceton und Acetonitril wobei die Verwendung von Aceton erfindungsgemäß besonders bevorzugt ist.

Gegebenenfalls kann es sinnvoll sein, die Umsetzung der Verbindung der Formel **1** mit **5** durch Zusatz eines Katalysators zu aktivieren. Besonders schonende Aktivierung ist erfindungsgemäß möglich durch Katalysatoren, die ausgewählt sind aus der Gruppe bestehend aus Zeolithe , Lipasen, tert. Amine, wie beispielsweise N,N-Dialkylaminopyridin, 1,4-Diazabicyclo[2.2.2]octan (DABCO) und Diisopropylethylamin und Alkoholaten, wie beispielsweise, Natrium- oder Kalium-tert.-butylat, Natrium- oder Kalium-isopropylat oder Natrium- oder Kalium-ethylat, wobei die Verwendung von Zeolithen und insbesondere Zeolithen und Kalium-tert.-butylat, erfindungsgemäß besonders bevorzugt ist. Besonders bevorzugte Zeolithe sind Molekularsiebe die ausgewählt sind aus der Gruppe der Molekularsieben mit basischem Charakter bestehend aus Natrium-oder Kalium-haltigen Alumosilikaten, bevorzugt Molsiebe mit der Summenformel Na₁₂[(AlO₂)₁₂(SiO₂)₁₂] x H₂O, wobei die Verwendung von Molsieb Typ 4A (steht für Porengröße von 4 Angström) erfindungsgemäß besonders bevorzugt ist.

Die Umsetzung von **1** mit **5** zur Verbindung der Formel **6** kann , abhängig vom Katalysator-Typ bei erhöhter Temperatur erfolgen. Bevorzugt erfolgt die Umsetzung bei einer Temperatur von 30°C, besonders bevorzugt in einem Bereich von 0 bis 30°C.

Die Verbindungen der Formel **5** können nach im Stand der Technik bekannten Verfahren erhalten werden. Hierbei sei beispielsweise auf die WO03/057694 verwiesen, auf die an dieser Stelle diesbezüglich vollinhaltlich Bezug genommen wird.

Ein weiterer Aspekt der vorliegenden Erfindung betrifft die Verwendung von Verbindungen der Formel **2** als Ausgangsverbindungen zur Herstellung von Verbindungen der Formel **4**. Ein weiterer Aspekt der vorliegenden Erfindung betrifft die Verwendung von Verbindungen der Formel **2** als Ausgangsverbindungen zur Herstellung von Verbindungen der Formel **6**.

Ein weiterer Aspekt der vorliegenden Erfindung betrifft ein Verfahren zur Herstellung von Verbindungen der Formel **4**, dadurch gekennzeichnet, dass eine Verbindung der Formel **2** als Ausgangsverbindung zur Herstellung von Verbindungen der Formel **4** eingesetzt wird. Ein weiterer Aspekt der vorliegenden Erfindung betrifft ein Verfahren zur Herstellung von Verbindungen der Formel **6**, dadurch gekennzeichnet, dass eine Verbindung der Formel **2** als Ausgangsverbindung zur Herstellung von Verbindungen der Formel **6** eingesetzt wird.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken.

### Beispiel 1: N-Methylscopinium hexafluorophosphat

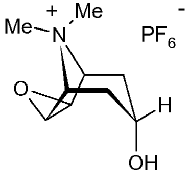

### Variante 1 a:

N-Methylscopolaminium bromid wird in Wasser gelöst unter Zusatz einer equimolaren Menge Kalilauge bei Raumtemperatur verseift und mit einer equimolaren oder molar überschüssigen Menge eines in Wasser löslichen Hexafluorophosphates (Natrium- oder Kaliumsalz) versetzt. Das N-Methylscopinium hexafluorophosphat kristallisiert als weißes, wenig wasserlösliches Produkt aus, wird isoliert, ggf. mit Methanol gewaschen und dann bei ca. 40°C im Vakuum getrocknet.

### Variante 1 b:

N-Methylscopolaminium bromid (40 g) wird in Methanol (120 ml) gelöst unter Zusatz einer katalytischen Menge ( 4-14 mol% ) Natriummethylat oder NaOH oder konz. Natronlauge zur Umesterungsreaktion gebracht (20-45°C) und anschließend mit einer equimolaren oder molar überschüssigen Menge einer Lösung aus Natriumhexafluorophosphat (18 g in 40 ml) Methanol versetzt.

Das N-Methylscopinium hexafluorophosphat fällt / kristallisiert als weißes, wenig wasserlösliches Produkt aus, wird isoliert, ggf. mit Methanol gewaschen und dann bei ca. 40°C im Vakuum getrocknet.
Ausbeute : 88-95%
Schmp.: 265-267°C (Schmelze unter Verfärbung);
H-NMR: in Acetonitril-d3 σ(ppm): 1,9 (dd, 2H), 2,55( dd, 2H), 2,9 (s,3H), 3,29 (s,3H), 3,95(dd, 4H), 3,85 (s, 1H).

### Beispiel 2: Tiotropiumbromid

1,6 g (5mmol) Methylscopinium hexafluorophosphat (Beispiel 1) und 2,0 g (7,8 mmol) Dithienylglycolsäuremethylester werden in 50 ml Aceton und in Gegenwart von 10g Molsieb 4A 50-70 Stunden unter Rückfluß gekocht.
Das Reaktionsgemisch wird filtriert, das Filtrat mit einer Lösung aus 0,3 g LiBr in 10 ml Aceton versetzt. Das auskristallisierte noch nicht umgesetzte N-Methylscopinium bromid wird durch Filtration abgetrennt. Nach Zugabe von weiteren 0,6 g LiBr (gelöst in Aceton) fällt Tiotropiumbromid in einer isolierten Ausbeute von 30% (bezogen auf eingesetzte Verbindung nach Beispiel 1) aus.

### Beispiel 3: Tiotropium hexafluorophosphat

Tiotropium hexafluorophosphat wird im Rahmen der Umsetzung nach Beispiel 2 nicht isoliert sondern direkt weiter zum Tiotropium bromid umgesetzt.
Für die Zwecke der Charakterisierung des Tiotropium hexafluorophosphat wurde dieses spezifisch hergestellt und isoliert. Hierbei wurden die folgenden charakterisierenden Daten erhalten. Schmp.: 233-236°C (Schmelzen unter Verfärbung)
H-NMR: in aceton-d6 : σ(ppm): 2,08 (dd, 2H), 2,23( dd, 2H), 3,32 (s,3H), 3,50 (s,3H), 3,62(s,2H), 4,28(m, 2H), 5,39(m, 1H) ,6,25 (s), 7,02(m,2H), 7,027,22(m,2H), 7,46(m,2H), P-NMR: in aceton-d6 : σ(ppm): -143,04, Heptett, J =4,37.

### Beispiel 4: Tiotropium bromid

31,5 g (100mol) Methylscopinium Hexafluorophosphat (Beispiel 1) und 25,4 g (100 mmol) Dithienylglycolsäuremethylester werden in 400 ml Aceton und in Gegenwart von 40g Molsieb 4A, Pulver (Fluka) und DMAP (4,4-Dimethylaminopyridin) 24h unter Rückfluß gekocht. (Molsieb wurde nach 3h in gleicher Menge ausgetauscht.)
Das Reaktionsgemisch wird filtriert, mit 200ml Aceton nachgewaschen, das Filtrat schrittweise mit einer Lösung aus 9,6 g LiBr (110mmol) in 110 ml Aceton versetzt. Das auskristallisierte noch nicht umgesetzte N-Methylscopinium bromid wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Tiotropiumbromid in einer isolierten Ausbeute von 16,6g (35%) (bezogen auf eingesetzte Verbindung nach Beispiel 1) aus. Reinheit HPLC> 99%. Reinheit DC: keine Verunreinigungen erkennbar.

### Beispiel 5: Tiotropiumbromid

1,6 g (5 mmol) Methylscopinium Hexafluorophosphat (Beispiel 1) und 1,25 g (5 mmol) Dithienylglycolsäuremethylester werden in 50 ml Aceton und in Gegenwart von 2g Molsieb 4A, Pulver (Fluka) und 6mg Kalium-tert.-butylat bei 0°C 4 h lang gerührt.
Das Reaktionsgemisch wird filtriert, mit 20ml Aceton nachgewaschen, das Filtrat schrittweise mit einer Lösung aus 0,7 g LiBr (13mmol) in 11 ml Aceton versetzt. Das auskristallisierte noch nicht umgesetzte wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Die Tiotropiumbromid-Fraktionen wurden abgesaugt, mit Aceton nachgewaschen, aus Wasser umkristallisiert, mit Aceton nachgewaschen und getrocknet. 1,2g (48% Ausbeute bezogen auf eingesetzte Verbindung nach Beispiel 1) Tiotropiumbromid wurden auf diese Weise isoliert.
Reinheit HPLC: 99,8% , DC: keine Verunreinigung sichtbar;

### Beispiel 6: Tiotropium bromid

31,5g (0,1 mol) Methylscopinium hexafluorophosphat (Beispiel 1) und 30,5g (0,10mol) 2,2'-Dithienylglycolsäuremethylester werden in 400 ml Aceton gelöst und in Gegenwart von 90g Zeolith vom Typ 4A (Na₁₂Al₁₂Si₁₂O₄₈ x n H₂O und 0,2g (1mmol) Kalium-tert.-butylat über einen Zeitraum von 20-24 Stunden bei 0°C gerührt.
Das Reaktionsgemisch wird filtriert, das Filtrat mit einer Lösung aus 8,7 g LiBr (8,7 g 0,10mol in 100 ml Aceton) versetzt.
Das dabei auskristallisierte Produkt wird durch Filtration abgetrennt, mit Aceton gewaschen und dann getrocknet.
Man erhält 41,4 g (87,7%) Ausbeute, bei 90% Umsetzungsgrad.

### Beispiel 7: N-Methylscopinium tetraphenylboranat

20g (80 mmol) Methylscopinium bromid werden in 500 ml Methanol gelöst.
27,38 (80mmol) Natriumtetraphenylboranat, gelöst in 150 ml Methanol werden zudosiert. Die entstandene Suspension wird 10 min bei Raumtemperatur nachgerührt und filtriert. Die abgetrennten Kristalle werden mit 50 ml Methanol gewaschen und getrocknet. Ausbeute: 39,1g (91,73% Ausbeute); Schmp.: 261°C.

### Beispiel 8: Tiotropium Tetraphenylboranat

0,245 g ( 0,5 mmol ) Methylscopinium tetraphenylboranat (Beispiel 7), und 0,154 g ( 0,6 mmol ) 2,2- Dithienylglycolsäuremethylester werden in 25 ml Aceton gelöst und in Gegenwart von 1,0 g Zeolith vom Typ 4A (Na₁₂Al₁₂Si₁₂O₄₈ x n H₂O) und 5 mg Kaliumtert.-butylat über einen Zeitraum von 20-30 Stunden bei 0°C gerührt.
Laut HPLC sind 79% des umgesetzten 2,2- Dithienylglycolsäuremethylesters nach 26 h in Tiotropium tetraphenylboranat verwandelt. (nicht isolierte Ausbeute: 43%).

Die beispielhaft aufgeführten Reaktionen erfolgen praktisch ohne Bildung von Nebenprodukten.

## Patentansprüche

1. Verfahren zur Herstellung von Scopiniumsalzen der Formel **1** 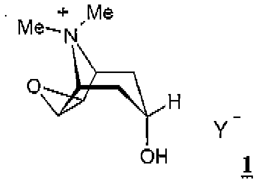 worin
Y⁻ ein einfach negativ geladenes lipophiles Anion ausgewählt aus der Gruppe bestehend aus Hexafluorophosphat, Tetrafluoroboranat, Tetraphenylboranat und Saccharinat, bevorzugt Hexafluorophosphat oder Tetraphenylboranat
**dadurch gekennzeichnet, daß** eine Verbindung der Formel **2** 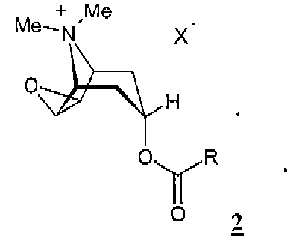 worin
X⁻ ein einfach negativ geladenes Anion ausgewählt aus der Gruppe bestehend aus Chlorid, Bromid, Iodid, Methansulfonat, p-Toluolsulfonat, Nitrat und Trifluormethansulfonat, bevorzugt Chlorid, Bromid, Iodid, Methansulfonat, Nitrat oder Trifluormethansulfonat, besonders bevorzugt Chlorid, Bromid oder Methansulfonat besonders bevorzugt Bromid; und
R ein Rest ausgewählt aus C₁-C₄-Alkyl, C₂-C₆-Alkenyl und C₁-C₄-alkylen-Phenyl, die jeweils substituiert sein können durch Hydroxy, Hydroxymethyl oder C₁-C₄-Alkoxy, bedeuten,
gegebenenfalls in Form ihrer Solvate oder Hydrate in einem geeigneten Lösemittel unter Zusatz einer geeigneten Base zunächst zu einer Verbindung der Formel **3** 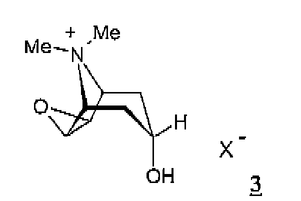 worin X⁻ die vorstehend genannten Bedeutungen haben kann, verseift wird und die Verbindung der Formel **3** ohne Isolierung durch Umsetzung mit einem Salz Kat⁺Y⁻, wobei Kat⁺ für ein Kation ausgewählt aus der Gruppe bestehend aus Li⁺, Na⁺, K⁺, Mg2+, Ca2+, steht und Y⁻ die vorstehend genannten Bedeutungen haben kann, in die Verbindung der Formel **1** überführt wird.

2. Verfahren nach Anspruch 1, **dadurch gekennzeichnet, dass** die Umsetzung mit einer Verbindung der Formel **2** erfolgt, in der X⁻ die in Anspruch 1 genannten Bedeutungen haben und kann und in der
R ein Rest ausgewählt aus -CH₃, -CH₂-CH₃, -CH₂-CH₂-OH, -CH(OH)-CH₃, -CH₂-Phenyl, -CH(OH)-Phenyl und -CH(CH₂OH)-Phenyl, bevorzugt -CH₃, -CH₂-CH₃, -CH₂-Phenyl, und -CH(CH₂OH)-Phenyl, besonders bevorzugt -CH(CH₂OH)-Phenyl, bedeutet.

3. Verfahren nach Anspruch 1 oder 2, worin Y - Hexafluorophosphat bedeutet.

4. Verwendung von Verbindungen der Formel **2** nach Anspruch 1 oder 2 als Ausgangsverbindungen zur Herstellung von Verbindungen der Formel **1**.

5. Verfahren zur Herstellung von Verbindungen der Formel **1 dadurch gekennzeichnet, dass** eine Verbindung der Formel **2** als Ausgangsverbindung eingesetzt wird.

6. Verfahren zur Herstellung von Tiotropiumsalzen der Formel **4** 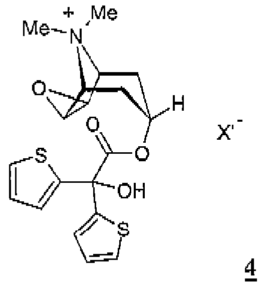 worin
X'⁻ ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat, p-Toluolsulfonat und Trifluormethansulfonat, bedeuten kann,
**dadurch gekennzeichnet, daß** eine Verbindung der Formel **2** 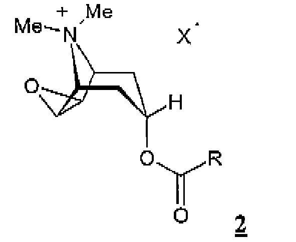 worin
X⁻ ein einfach negativ geladenes Anion ausgewählt aus der Gruppe bestehend aus Chlorid, Bromid, Iodid, Methansulfonat, p-Toluolsulfonat, Nitrat und Trifluormethansulfonat, bevorzugt Chlorid, Bromid, Iodid, Methansulfonat, Nitrat oder Trifluormethansulfonat, besonders bevorzugt Chlorid, Bromid oder Methansulfonat, besonders bevorzugt Bromid; und
R ein Rest ausgewählt aus C₁-C₄-Alkyl, C₂-C₆-Alkenyl und C₁-C₄-alkylen-Phenyl, die jeweils substituiert sein können durch Hydroxy, Hydroxymethyl oder C₁-C₄-Alkoxy, bedeuten,
gegebenenfalls in Form ihrer Solvate oder Hydrate in einem geeigneten Lösemittel unter Zusatz einer geeigneten Base zunächst zu einer Verbindung der Formel **3** 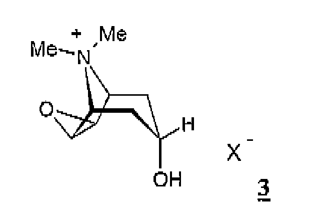 worin X⁻ die vorstehend genannten Bedeutungen haben kann, verseift wird und die Verbindung der Formel **3** ohne Isolierung durch Umsetzung mit einem Salz Kat⁺Y⁻, wobei Kat⁺ für ein Kation ausgewählt aus der Gruppe bestehend aus Li⁺, Na⁺, K⁺, Mg2+, Ca2+, steht und
Y⁻ ein einfach negativ geladenes lipophiles Anion, vorzugsweise ein Anion ausgewählt aus der Gruppe bestehend aus Hexafluorophosphat, Tetrafluoroboranat, Tetraphenylboranat und Saccharinat, besonders bevorzugt Hexafluorophosphat oder Tetraphenylboranat
bedeuten kann, in die Verbindung der Formel **1** 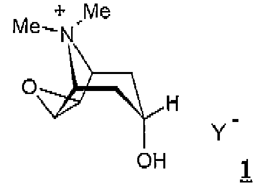 worin Y⁻ die vorstehend genannten Bedeutungen haben kann, überführt und anschließend die Verbindung der Formel **1** in einem Schritt mit einer Verbindung der Formel **5** 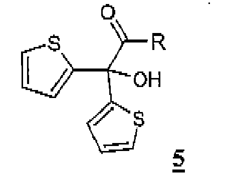 worin
R ein Rest ausgewählt aus der Gruppe bestehend aus Methoxy, Ethoxy, Propoxy, Isopropoxy, Isopropenyloxy, Butoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet,
in einem geeigneten Lösemittel zu einer Verbindung der Formel **6** 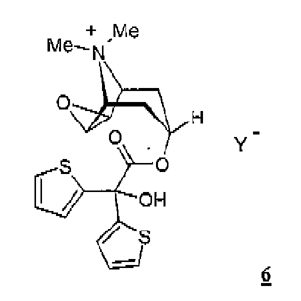 umgesetzt wird, wobei die Gruppe Y⁻ die vorstehend genannten Bedeutungen haben kann, und die Verbindung der Formel **6** ohne Isolierung durch Umsetzung mit einem Salz Kat⁺X'⁻, wobei Kat⁺ für ein Kation ausgewählt aus der Gruppe bestehend aus Li⁺, Na⁻, K⁺, Mag2+, Ca2+, organische Kationen mit quartärem N steht und X'⁻ die vorstehend genannten Bedeutungen haben kann, in die Verbindung der Formel **4** überführt wird.

7. Verwendung von Verbindungen der Formel **2** als Ansgangsverbindungen zur Herstellung von Verbindungen der Formel **4**.

8. Verwendung von Verbindungen der Formel **2** als Ausgangsverbindungen zur Herstellung von Verbindungen der Formel **6**.

## Claims

1. Process for preparing scopinium salts of formula **1** 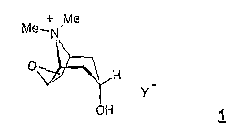 wherein
Y⁻ denotes a lipophilic anion with a single negative charge selected from among hexafluorophosphate, tetrafluoroboranate, tetraphenylboranate and saccharinate, preferably hexafluorophosphate or tetraphenylboranate
**characterised in that** a compound of formula **2** 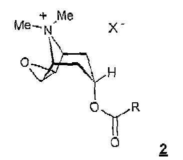 wherein
X⁻ denotes an anion with a single negative charge selected from among chloride, bromide, iodide, methanesulphonate, p-toluenesulphonate, nitrate and trifluoromethanesulphonate, preferably chloride, bromide, iodide, methanesulphonate, nitrate or trifluoromethanesulphonate, particularly preferably chloride, bromide or methanesulphonate, particularly preferably bromide; and
R denotes a group selected from C₁-C₄-alkyl, C₂-C₆-alkenyl and C₁-C₄-alkylene-phenyl, which may be substituted in each case by hydroxy, hydroxymethyl or C₁-C₄-alkoxy.
optionally in the form of the solvates or hydrates thereof, is saponified in a suitable solvent with the addition of a suitable base to form initially a compound of formula **3** 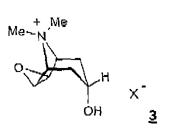 wherein X may have the meanings given above, and the compound of formula **3** without being isolated is converted into the compound of formula **1** by reacting with a salt Cat⁺Y⁻, wherein Cat⁺ denotes a cation selected from among Li⁺, Na⁺, K⁺, Mg²⁺, Ca²⁺, and Y⁻ may have the meanings given above.

2. Process according to claim 1, **characterised in that** the reaction is carried out with a compound of formula **2**, wherein X⁻ may have the meanings given in claim 1 and wherein
R denotes a group selected from -CH₃, -CH₂-CH₃, -CH₂-CH₂-OH, -CH(OH)-CH₃, -CH₂-phenyl, -CH(OH)-phenyl and -CH(CH₂OH)-phenyl, preferably-CH₃, -CH₂-CH₃, -CH₂-phenyl, and -CH(CH₂OH)-phenyl, particularly preferably -OH(CH₂OH)-phenyl.

3. Process according to claim 1 or 2, wherein Y⁻ denotes hexafluorophosphate.

4. Use of compounds of formula **2** according to claim 1 or 2 as starting compounds for preparing compounds of formula **1**.

5. Process for preparing compounds of formula **1**, **characterised in that** a compound of formula **2** is used as the starting compound.

6. Process for preparing tiotropium salts of formula **4** 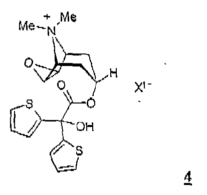 wherein
X'⁻ may denote an anion with a single negative charge, preferably an anion selected from among chloride, bromide, iodide, sulphate, phosphate, methanesulphonate, nitrate, maleate, acetate, citrate, fumarate, tartrate, oxalate, succinate, benzoate, p-toluenesulphonate and trifluoromethanesulphonate,
**characterised in that** a compound of formula **2** 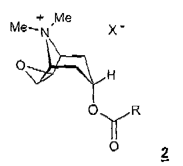 wherein
X⁻ denotes an anion with a single negative charge selected from among chloride, bromide, iodide, methanesulphonate, p-toluenesolphonate, nitrate and trifluoromethanesulphonate, preferably chloride, bromide, iodide, mathanesulphonate, nitrate or trifluoromethanesulphonate, particularly preferably chloride, bromide or methanesulphonate, particularly preferably bromide; and
R denotes a group selected from C₁-C₄-alkyl, C₂-C₆-alkenyl and C₁-C₄-alkylene-phenyl, which may be substituted in each case by hydroxy, hydroxymethyl or C₁-C₄-alkoxy,
optionally in the form of the solvates or hydrates thereof, is saponified in a suitable solvent with the addition of a suitable base to form initially a compound of formula **3** 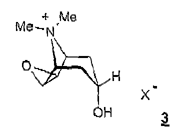 wherein X⁻ may have the meanings given above, and the compound of formula **3** without being isolated is converted, by reaction with a salt Cat⁺Y⁻, wherein Cat⁺ denotes a cation selected from among Li⁺, Na⁺, K⁺, Mg²⁺, Ca²⁺, and
Y⁻ denotes a lipophilic anion with a single negative charge, preferably an anion selected from among hexafluorophosphate, tetrafluoroboranate, tetraphenylboranate and saccharinate, particularly preferably hexafluorophosphate or tetraphenylboranate
into the compound of formula **1** 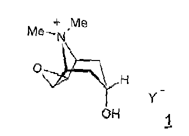 wherein Y⁻ may have the meanings given above, and then the compound of formula **1** is reacted in one step with a compound of formula **5** 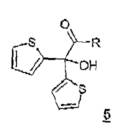 wherein
R denotes a group selected from among methoxy, ethoxy, propoxy, isopropoxy, isopropenyloxy, butoxy, O-N-succinimide, O-N-phthalimide, phenyloxy, nitrophenyloxy, fluorophenyloxy, pentafluorophenyloxy, vinyloxy, 2-allyloxy, -S-methyl, -S-ethyl and -S-phenyl,
in a suitable solvent to form a compound of formula **6** 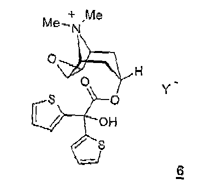 wherein the group Y⁻ may have the meanings given above, and the compound of formula 6 without being isolated is converted into the compound of formula 4 by reaction with a salt Cat⁺X'⁻, wherein Cat⁺ denotes a cation selected from among Li⁺, Na⁺, K⁺, Mg²⁺, Ca²⁺, organic cations with quaternary N and X'⁻ may have the meanings given above.

7. Use of compounds of formula 2 as starting compounds for preparing compounds of formula 4.

8. Use of compounds of formula 2 as starting compounds for preparing compounds of formula 6.

## Revendications

1. Procédé de production de sels de scopinium de
formule 1 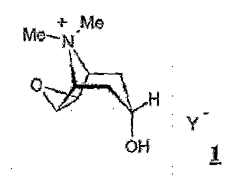 dans laquelle
Y⁻ désigne un anion lipophile à charge négative simple choisi dans le groupe comprenant l'hexafluorophosphate, la tétrafluoroboranate, le tétraphénylboranate et le saccharinate,
de préférence, le hexafluorophosphate ou le tétraphénylboranate
**caractérisé en ce qu'**un composé de formule 2 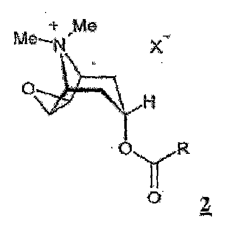 dans laquelle
X⁻ désigne un anion à charge négative simple choisi dans le groupe comprenant le chlorure, le bromure, l'iodure, le méthanesulfonate, le p-toluènesulfonate, le nitrate et le trifluorométhanesulfonate, de préférence, le chlorure, le bromure, l'iodure, le méthanesulfonate, le nitrate ou le trifluorométhanesulfonate, de manière particulièrement préférée, le chlorure, le bromure ou le méthanesulfonate, de manière particulièrement préférée, le bromure ; et
R désigne un radical choisi parmi les groupes alkyle en C₁ à C₄, alcényle en C₂ à C₆ et alkylène en C₁ à C₄-phényle, qui peuvent être substitués respectivement par un groupe hydroxy, hydroxyméthyle ou alcoxy en C₁ à C₄,
éventuellement sous la forme de leurs solvates ou leurs hydrates, dans un solvant approprié en ajoutant une base appropriée, est saponifié tout d'abord en un composé de formule 3 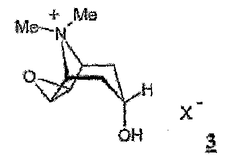 dans laquelle X⁻ peut avoir les significations indiquées précédemment, et le composé de formule 3 est converti, sans isolement, par réaction avec un sel cat⁺Y⁺, où cat⁺ désigne un cation choisi dans le groupe comprenant Li⁺, Na⁺, K⁺, Mg2⁺, Ca2⁺ et Y⁻ peut avoir les significations mentionnées précédemment, en composé de formule 1.

2. Procédé selon la revendication 1, **caractérisé en ce que** la réaction s'effectue avec un composé de formule 2, dans laquelle X⁻ peut avoir les significations mentionées dans la revendication 1 et dans laquelle
R designe un radical choisi parmi -CH₃, -CH₂-CH₃, - CH₂-CH₂-OH, -CH(OH)-CH₃, -CH₂-phényle, -CH(OH)-phényle et -CH(CH₂OH)-phényle, de préférence, -CH₃, -CH₂-CH₃, -CH₂-phényle et -CH(CH₂OH)-phényle, de manière particulièrement préférée, -CH(CH₂OH)-phényle.

3. Procédé selon la revendication 1 ou 2, dans lequel Y⁻ désigne l'hexafluorophosphate.

4. Utilisation de composés de formule 2 selon la revendication 1 ou 2 comme composés de départ pour la production de composés de formula 1.

5. Procédé de production de composés de formule 1, **caractérisé en ce qu'**un composé de formule 2 est utilisé comme composé de départ.

6. Procédé de production de sels de tiotropium de formule 4 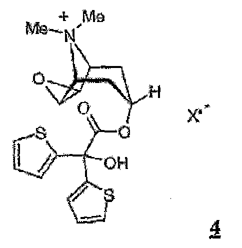 dans laquelle
X'⁻ peut désigner un anion à charge simplement négative, de préférence un anion choisi dans le groupe comprenant le chlorure, le bromure, l'iodure, le sulfate, le phosphate, le méthanesulfonate, le nitrate, le maléate, l'acétate, le citrate, le fumarate, le tartrate, l'oxalate, le succinate, le benzoate, le p-toluène-sulfonate et le trifluorométhanesulfonate,
**caractérisé en ce qu'**un composé de formule 2 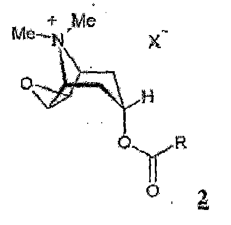 dans laquelle
X⁻ désigne un anion à charge négative simple choisi dans le groupe comprenant le chlorure, le bromure, l'iodure, le méthanesulfonate, le p-toluènesulfonate, le nitrate et le trifluoro-méthenesulfonate, de préférence, le chlorure, le bromure, l'iodure, le méthanesulfonate, le nitrate ou le trifluorométhanesulfonate, de manière particulièrement préférée, le chlorure, le bromure ou le méthanesulfonate, de manière particulièrement préférée, le bromure; et
R désigne un radical choisi parmi les groupes alkyle en C₁ à C₄, alcényle en C₂ à C₆ et alkylène en C₁ à C₄-phényle, qui peuvent être substitués respectivement par un groupe hydroxy, hydroxyméthyle ou alcoxy en C₁ à C₄,
éventuellement sous la forme de leurs solvates ou leurs hydrates, dans un solvant approprié en ajoutant une base appropriée, est saponifié tout d'abord en composé de formule 3 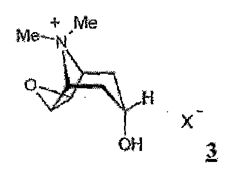 dans laquelle X⁻ peut avoir les significations indiquées précédemment, et le composé de formule 3 est converti, sans isolement, par réaction avec un sel cat⁺Y⁺, où cat⁺ désigne un cation choisi dans le groupe comprenant Li⁺, Na⁺, K⁺, Mg²⁺, Ca²⁺ et
Y⁻ peut désigner un anion lipophile à charge négative simple, de préférence un anion choisi dans le groupe comprenant l'hexafluorophosphate, le tétrafluorophosphate, le tétraphénylboranate et le saccharinate, de manière particulièrement préférée, l'hexafluorophosphate ou le tétraphénylboranate,
en composé de formule 1 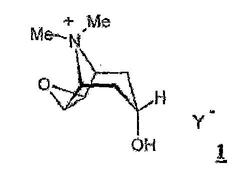 dans laquelle Y⁻ peut avoir les significations mentionnées précédemment, et ensuite on fait réagir le
composé de formule 1 en une étape avec un composé de formule 5 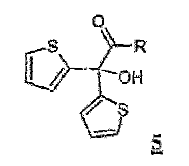 dans laquelle
R désigne un radical choisi dans le groupe comprenant un groupe méthoxy, éthoxy, propoxy, isopropoxy, isopropényloxy, butoxy, O-N-succinimide, O-N-phtalimide, phényloxy, nitrophényloxy, fluorophényloxy, pentafluoro-phényloxy, vinyloxy, 2-allyloxy, -S-méthyle, -S-éthyle et -S-phényle,
dans un solvant approprié, en un composé de formule 6 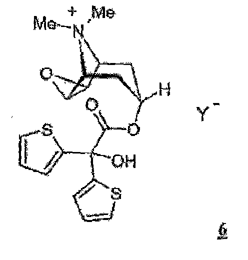 dans laquelle le groupe Y⁻ peut avoir les significations mentionnées précédemment, et le composé de formule 6 est converti sans isolement, par réaction avec un sel cat ⁺X'⁻, où cat⁺ désigne un cation choisi dans le groupe comprenant Li⁺, Na⁺, K⁺, Mg²⁺, Ca²⁺, des cations organiques avec un N quaternaire et X'⁻ peut avoir les significations mentionnées précédemment, en composé de formule 4.

7. Utilisation de composés de formule 2 comme composés de départ pour la production de composés de formule 4.

8. Utilisation de composés de formule 2 comme composés de départ pour la production de composés de formule 6.
